# EUROPEAN PATENT APPLICATION

(11) **EP 2 077 320 A1**
(43) Date of publication of application: **08.07.2009**
(21) Application number: 07829204.2
(22) Date of filing: 04.10.2007
(51) Int. Cl.: C12N 5/06, C12N 1/04

(54) **METHOD FOR FREEZE PRESERVATION OF TISSUE-DERIVED CELL**

(30) Priority: 05.10.2006 JP 2006274243
(71) Applicant: Cell Bank, Corp., Tokyo 108-0073 (JP)
(72) Inventor: HOJO, Motoharu, Yokohama-shi, Kanagawa 225-0002 (JP)
(74) Representative: Arends, William Gerrit
(86) International application number: PCT/JP2007/069466
(87) International publication number: WO 2008/041744

(57) **Abstract**

An object of the present invention is to provide a method for cryopreservation of desired cells derived from various tissues simply and effectively. The method comprises: dividing a tissue containing cells of interest into pieces; culturing the divided tissue pieces in a predetermined culture medium; collecting the cultured tissue pieces; suspending the cultured tissue pieces in a cryoprotective solution; and freezing the resulting suspension of the tissue pieces at a temperature of -70°C or lower. In a preferred mode, the cells are fibroblasts derived from skin tissue.

## Description

### TECHNICAL FIELD

The present invention relates to a method for freeze preservation (cryopreservation) of tissue-derived cells, and more specifically, the present invention relates to a method for cryopreservation of desired cells derived from various mammalian tissues simply and effectively.
This application claims priority based on Japanese Patent Application No. 2006-274243 filed on October 5, 2006, the disclosure of which is incorporated herein by reference thereto.

### BACKGROUND ART

The skin or organ of a mammal is used as a transplantation tissue for filling a patient's defective site, or for examining the effect of medicines or cosmetics after sampling from a subject as it is or after subsequent culturing and proliferation. On the other hand, intense interest has been shown towards so-called application to regenerative medicine in which stem cells are extracted from a specific tissue and are used after proliferation. Since there is a limitation on the shelf life of a tissue collected from a living body or a cultured tissue in a state of maintaining the function, it is impossible to preserve proper stocks over all the length of period of time. Cryopreservation of the tissue and cells is carried out so as to prolong the storage period. As a technique used when the tissue is preserved, there are disclosed a technique in which a cultured tissue is freeze-preserved by immersing in a preserving solution containing a cryoprotective agent added therein (for example, refer to Patent Document 1) and a technique of preserving at a low temperature of 8 to 19°C in a state where a cultured epithelium sheet is immersed in DMEM (for example, refer to Patent Document 2), or the like.

On the other hand, as a method for preservation of mammal cells, there has been established a method in which freeze preservation is carried out so as to minimize genetic change in the case of established cells or to avoid aging and transformation in the case of cells having a given lifetime (for example, refer to Non-Patent Documents 1 and 2). According to this method, in order to preserve cells such as cultured cells so as to retain 50% or more of activity, cells are gradually cooled at a rate of about 1°C per minute, preserved at about -200°C and quickly fused (thawed) after completion of the preservation. In order to prevent cytoclasis due to freezing, cells are suspended in a complete medium containing 10% glycerol, or a condition medium or serum-free medium containing 5-10% dimethyl sulfoxide. Freezing may cause a problem that a liquid is crystallized upon solidification to form ice crystal in cells. The cryohydrates exert an adverse influence on viability of the cells upon thawing. It is considered that aforementioned cryoprotective agent such as glycerol causes vitrification or amorphization of the liquid in the cells thereby suppressing formation of ice crystal.

For obtaining cells from a mammal tissue, there is a method in which cells are dispersed by using enzyme/enzyme treatment etc. followed by culturing or a tissue itself is immersed in a culture medium and cells migrated into the culture medium from the tissue are obtained or the like. All the documents cited in the specification are incorporated herein by reference thereto.

Patent Document 1: Japanese Kohyo Patent Publication JP-A-9-505032 of the PCT Application
Patent Document 2: Japanese Patent No. 2,693,640
Non-Patent Document 1: Toshinori Ide, Saibo-Baiyo Nyumon Nohto (Cell Culture Introduction Note), YODOSHA CO., LTD., p139
Non-Patent Document 2: Shiguma-Saibo Baiyo Manyuaru (Sigma Cell Culture Manual), 2005, SIGMA-ALDRICH Japan K.K., p286

### DISCLOSURE OF THE INVENTION

### Problems to be Solved by the Invention

When the sampled cells are collected after dispersing by enzyme treatment/using enzyme, it is difficult to collect only cells of interest since various cells exist in a mixed fashion. Therefore, considerable labor and time are required to culture the collected cells in a selection medium in which only specific cells are grown, followed by selection of cells of interest. In contrast, a method in which tissue pieces themselves are immersed in a culture medium and then cells gradually migrate into a culture medium from a tissue has a problem that much labor and time are required since culture over a long period of time and subculture for separation of the migrated cells from the tissue are necessary.

### Means for Solving the Problems

The present inventors have found that, when only the cells of interest migrate to the border or marginal area of tissue pieces from the sampled tissue and freeze-preserved as they are, cells in the tissue are severely damaged as a result of formation of ice or a crystal of a solute, whereas, cells existing at the border or marginal area of the tissue can be stored over a long period of time according to ordinary method of freezing without being damaged even when ice or a crystal of a solute is formed in the tissue, and also can be collected as viable cells by thawing the frozen tissue. Thus, the present invention has been completed based on this finding.

The method for cryopreservation of tissue-derived cells of the present invention comprises: dividing a tissue containing cells of interest into fragments/pieces; culturing the divided tissue piece(s) in a predetermined culture medium; collecting the cultured tissue piece(s); suspending the cultured tissue piece(s) in a cryoprotective solution; and freezing the resulting suspension of the tissue piece(s) at a temperature of -70°C or lower.

In a preferred embodiment of the present invention, the tissue is a tissue sampled (or taken) from a mammal, or a cultured tissue. The tissue pieces are cultured for a period which is required for cells in the tissue to migrate to the border or marginal area of the tissue piece(s). The predetermined culture medium is preferably a culture medium capable of migrating desired cells existing in the tissue to the border or marginal area of the tissue piece(s).

In one embodiment of the present invention, the method further comprises: thawing the frozen suspension of the tissue piece(s), culturing the thawed tissue piece(s), and collecting cells migrated in the culture medium.

In another embodiment of the present invention, there is provided a method for cryopreservation of dermis-derived fibroblasts, which comprises: dividing a skin sampled (or taken) from a mammal into fragments/pieces; culturing the divided skin piece(s) in a serum-containing minimal essential medium; collecting the cultured skin piece(s); suspending the cultured skin piece(s) in a cryoprotective solution; and freezing the resulting suspension of the skin piece(s) at a temperature of -70°C or lower. The divided skin pieces preferably have a maximum diameter of 5 mm or less, and more preferably 1 mm to 0.5 mm. The skin pieces are preferably cultured under conventional cell culture conditions, for example, conditions of 5% carbon dioxide in air at a temperature of about 37°C for 5 to 10 days. In one typical embodiment, skin pieces are cultured for about one week (7 days).

In a still preferred embodiment, the cryoprotective solution contains 10% glycerol or 5-10% dimethyl sulfoxide, and a suspension of the tissue pieces is frozen in liquid nitrogen.

### Effects of the Invention

Since the tissue pieces frozen by the method of the present invention contain viable cells originating from the tissue, a lot of living cells can be collected when the tissue pieces are thawed and cultured. Therefore, cryopreservation can be completed within a short period of time, for example, about one week in the case of skin fibroblasts, when compared with the case where a tissue sampled from the living body is cultured and only cells are preliminarily collected and preserved. Thus, it becomes possible to preserve tissue-originating cells within a short period of time at a low cost. By using a predetermined culture medium in culturing the tissue pieces before freezing, only the desired tissue-originating cells can be migrated to the border or marginal area of tissue piece(s) and preserved in a viable state. Therefore, only desired cells can be efficiently obtained when the tissue pieces are thawed and cultured after completion of cryopreservation.

### BRIEF DESCRIPTION OF THE DRAWINGS

Fig. 1 is a photograph showing views immediately after thawing when a skin tissue is directly freeze-preserved (group D) and when a skin tissue is freeze-preserved by the method of the present invention (group C).
Fig. 2 is a photograph showing views on day 1 after culture is started when a skin tissue is directly freeze-preserved (group D) and when a skin tissue is freeze-preserved by the method of the present invention (group C).
Fig. 3 is a photograph showing views on day 7 after culture is started when a skin tissue is directly freeze-preserved (group D) and when a skin tissue is freeze-preserved by the method of the present invention (group C).

### PREFFERRED MODES FOR CARRYING OUT THE INVENTION

### (Definition)

In this Description, terms shown below are defined as follows. The term "tissue" means a cell population having the same function and form as a result of differentiation of component cells in multicellular organism. The animal tissue can be roughly classified into an epithelial tissue, connective tissue, muscular tissue and nervous tissue based on morphological, functional or genetic basis. Components of the tissue include not only cells but also a matrix as a substance which fills the intercellular space. Plural tissues or cells constitute an organ. The animal organ is also called as viscus or viscera. In the present invention, the term "tissue" may also be used as the same meaning as that of "organ" or "viscera" and, for example, includes, in addition to skin, skeletal muscle, cardiac muscle and nerve, liver, kidney, pancreas, spleen, spermary, ovary, adrenalcapsul, brain and thyroid gland.

The term "cryoprotective solution" (or freeze preservation solution) means any protective agent (or cryoprotectant) for protection from damage on cells by crystallization of a liquid component when the tissue and cells are freeze-preserved, and includes cell-permeating vitrification agent and cell-nonpermeating vitrification agent. Examples of the cell-nonpermeating vitrification agent include chondroitin sulfate, polyvinyl pyrrolidone, polyethylene glycol, and high molecular weight formulation of a complex carbohydrate, such as hydroxyethyl starch. Examples of the cell-permeating vitrification agent include glycerol, propylene glycol, ethylene glycol and dimethyl sulfoxide (DMSO) etc. Examples of preferred cryoprotective solution include, but are not limited to, 10% glycerol-containing complete medium, 10% DMSO-containing complete medium, 10% glycerol-containing 50% condition medium + 50% fresh medium, 10% DMSO-containing 50% condition medium + 50% fresh medium, 7.5% DMSO-containing 50% condition medium + 50% serum-free medium, and fresh serum-free medium containing 7.5% DMSO and 10% BSA or the like.

The term "medium" or "culture solution" means a nutrient component which is fed so as to proliferate or grow cells, tissues and organs in vitro. The term "border or marginal area" means a terminal region of divided tissue pieces, which is within several micrometers from the end of the tissue pieces. The term "migration" means that cells transfer (migrate) from one place to another place in the tissue, organ or living body. It is a kind of migration that blood cells transfer (migrate) through vessel walls and also tumor cells transfer or spread.

### (Freeze Preservation of Tissue-Derived Cells)

In the method for cryopreservation of the present invention, a tissue sampled from the living body is divided (fragmented) first. A preferred division (or fragmentation) method can be used according to each tissue and, for example, any of a chemical method using enzyme(s) with trypsin or collagenaze etc. and a physical method of slicing using surgical scissors or scalpel may be used. A comparatively thin and weak tissue such as mammal skin is preferably divided by a physical method. The size of the divided tissue pieces (or fragments) varies depending on the tissue to be used and is preferably 5 mm or less, and more preferably from 1 mm to 0.5 mm, in terms of a maximum diameter in the case of a skin tissue.

Next, the divided tissue pieces are cultured in a predetermined culture medium. For example, divided mammal skin can be used. When human skin is viewed histologically, the skin is made up of epidermis, dermis and subcutaneous tissue in this order from the surface to form layers. The epidermis is mainly formed of keratinocytes, melanocytes, merkel cells which are considered to be involved in perception, and Langerhans cells which are antigen presenting cells and are bone marrow-derived cells. The epidermis is very thin (0.1 to 0.3 mm) and keratinocytes play a role of a breakwater in the epidermis. Mother cells existing in the lower layer surface skin repeatedly proliferate to form a chemically and physically tough cornified layer (in a state where the inside of cells is filled with hard keratin fibers) on the surface of the skin, and the cornified layer serves as a barrier. It is important for healing of wound to cover the surface of the wound as a result of migration of keratinocytes. In contrast, the dermis is made up of fibroblast cells and an elastic collagenous interstitium produced by these cells and imparts skin flexibility and elasticity, and also rich in capillary vessels and has a coordinate action with the epidermis. The dermis (collagen fibers, elastin fibers, fibroblasts) is protected beneath the epidermis and is not likely to be damaged from the outside, and also support a function of the skin by moisture retention and mainly play a role of maintaining skin elasticity and tenseness.

By adjusting the composition of a culture medium for culturing tissue pieces, and adding a serum or not or adjusting the amount of serum upon addition, desired cells existing in the tissue can be migrated to the border or marginal area of the tissue pieces. As one embodiment of the present invention, in the case of a skin tissue, dermis-derived fibroblasts can preferably migrate by using a serum-containing minimal essential medium. The minimal essential medium is a medium containing nutrient sources in the defined number which enable cell culturing, and examples thereof include Eagle's MEM medium, Dulbecco's modified Eagle's medium (DMEM), Ham's Medium and RPMI1640 medium etc. It is known that a serum-free medium having a low calcium concentration is used so as to suppress proliferation of fibroblasts and selectively migrating keratinocytes having proliferation capability. It is important to add a peptide additive (epidermal growth factor, insulin, basic fibroblast growth factor) to the medium so as to promote migration of keratinocytes and melanocytes.

As another embodiment, there is a report of a method for selective migration of Schwann cells which are useful as a material for inducing neurotization in peripheral nerve injury (refer to Japanese Kokai Patent Publication JP-A-2003-70465). According to this method, after enzymegenated peripheral nerve pieces are cultured in a serum-containing medium for a predetermined period of time and then cultured in a serum-free medium for a predetermined period of time, migration of Schwann cells can be induced under suppression of proliferation of figroblasts. The peripheral nerve pieces may also be cultured in a medium containing a low concentration of serum, for example, about 2.5% of FCS. In another embodiment, it is also possible to selectively migrate vascular endothelial cells or vascular smooth muscle cells from umbilical vein, arota and pulmonary artery etc. Media to be used herein are commercially available from distributors such as SIGMA Corp., ALDRICH Corp., INVITROGEN/GIBCO Corp., CLONTECH Corp., QIAGEN Corp., KURABO Corp. and TAKARA BIO INC etc.

The tissue pieces are preferably cultured for a required period of time so as to let desired cells migrate in the tissue to the border or marginal area of the tissue pieces. For example, in the case of a skin tissue, culturing is carried out at a culture temperature of about 37°C for 5 to 10 days, and preferably about one week (7 days) . When the culture period is short, dermis fibroblasts do not completely migrate to the border or marginal area of the skin tissue pieces and remain in the tissue (in the skin tissue pieces), and thus these cells are damaged when the tissue pieces are frozen. In contrast, when the culture period is longer than the above range, cells will migrate to the exterior of the tissue pieces and thus the effect to be exerted is not more than that of conventional cryopreservation.

Subsequently, the cultured tissue pieces are collected and then suspended in a cryoprotective solution after removing the culture medium. The cultured tissue pieces float in the medium or loosely adhere to a culture vessel and therefore can be easily collected by applying a slight impact to the vessel, or using a pipetting operation.

Finally, a suspension of the tissue pieces is frozen at a temperature of -70°C or lower. Preferably, the suspension is cooled to a temperature within a range from about -140°C to about -180°C. The cooling rate is preferably about -1°C per minute gradually. The suspension is frozen by using a program freezer, or frozen in a freezer at about -70°C to about -90°C after standing a vial in a heat insulating box (such as a styrene foam box). After freezing, the frozen suspension is preferably transferred to a liquid nitrogen preservation vessel and preserved at a still lower temperature.

### (Thawing of Freeze-Preserved Suspension of Tissue Pieces)

The frozen suspension of the tissue pieces is thawed by heating at a high speed so as to thaw within one to several seconds. For example, a tube for freezing the suspension of the tissue pieces may be heated in a water bath or induction-heated. Preferably, the suspension of the tissue pieces frozen in the tube is thawed by immersing in a water bath at 37°C.

Since cells may be damaged when contacted with a cryoprotective agent (cryoprotectant) for a long time depending on the kind of the cryoprotective agent, it is preferred to remove a cryoprotective agent solution from the tissue pieces within a period of time as short as possible so as to maintain vitality of cells existing at the border or marginal portion of the tissue pieces. For example, the contents of an ampoule or vial are transferred to a flask or proper vessel containing 15 to 20 ml of a culture medium and then incubated in a usual manner. In the case of adhesive cells, a culture medium containing a cryoprotective agent, which has already been diluted, is removed immediately after almost all of cells are adhered (for 3 to 4 hours, usually) and then replaced by a new culture medium. In the case of cells having sensitivity to a cryoprotective agent, the used culture medium can be easily removed by centrifugation at a low speed. For example, the contents of an ampoule or vial are transferred to a 15 ml centrifugal tube containing 15 ml of a new culture medium and centrifuged at 100 × g for 5 minutes. After discarding the supernatant containing the cryoprotective agent, pellets of the tissue pieces are resuspended in a new culture medium. Subsequently, a suspension of the tissue pieces are transferred to a proper culture vessel and then incubated in a usual manner.

Hereinafter, materials and methods used to carry out the present invention are described specifically with reference to the Examples, however, the present invention is not necessarily limited to the following Examples.

### Example 1

Skin was sampled from three female adults (aged 33, 27 and 35) and one male adult (aged 50) with the principal's approval, and then the following test was carried out. After anesthesia of each subject, full-thickness skin of 1 mm to 3 mm in thickness was sampled from the cranial surface of the auricle. Immediately, the full-thickness skin was subjected to enzymegenation (2,000 units/ml of DISPASE, manufactured by GODO SHUSEI CO., LTD.) for 2 hours and separated into an epidermis layer and a dermis layer. After discarding the epidermis layer, the dermis layer was finely cut into slices (measuring about 0.5 mm in diameter) to give 10 fragmental specimens (sections). The resulting 10 fragmental specimens were divided into two groups, which were subjected to the following test.

### Group D (Direct Group)

Fragmental specimens (sections of specimen) were immersed in a freeze preservation solution (10% DMSO, 80% α MEM, 10% human serum) or a commercially available freeze preservation solution CELLBANKER^{®} 1 (manufactured by JUJI FIELD INC.), stored at -80°C for 12 hours and then frozen in liquid nitrogen for 2 weeks.

### Group C (Culture Group)

Fragmental specimens were incubated in a α MEM medium containing 10% human serum under an atmosphere of 5% carbon dioxide in air at 37°C for a week. The fragmental specimens were collected in a centrifugal tube and the supernatant was removed. Subsequently, in the same manner as described above, the fragmental specimens were immersed in a freeze preservation solution, stored at -80°C for 12 hours and then frozen in liquid nitrogen for 2 weeks.

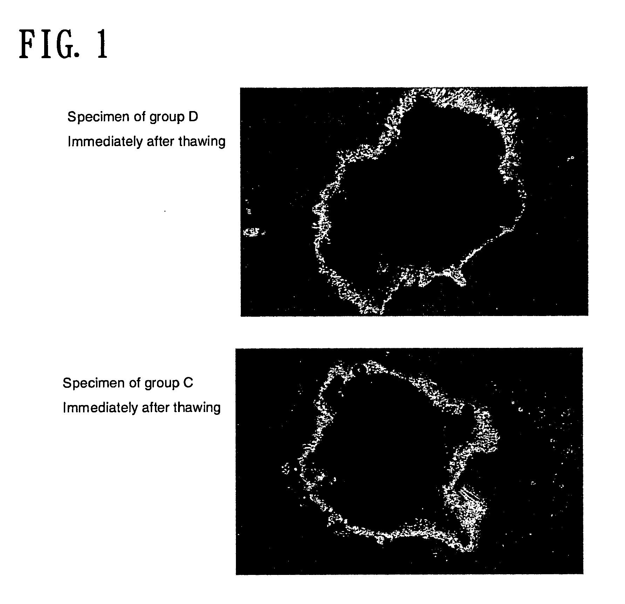

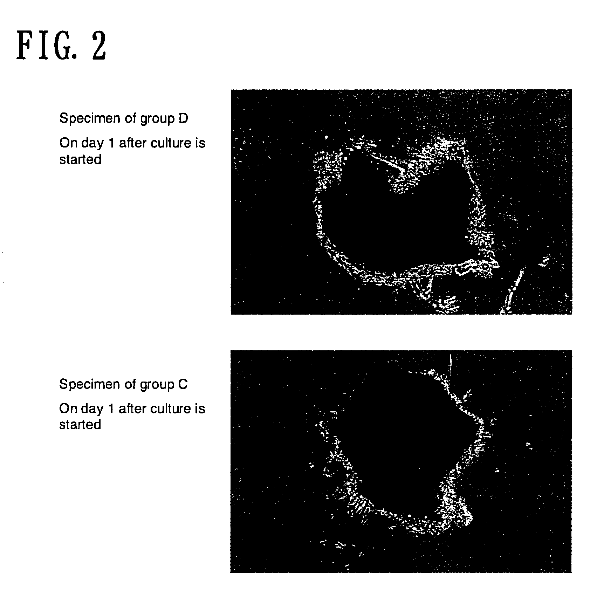

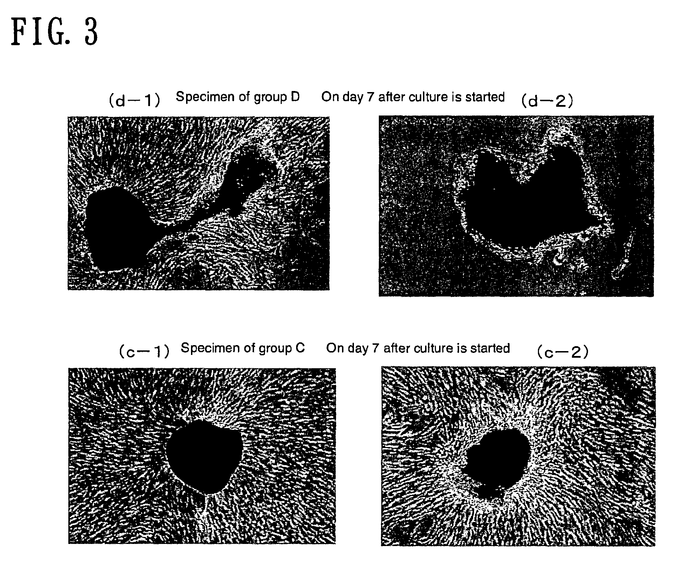

After 2 weeks, each specimen of the above groups D and C was taken out from liquid nitrogen, thawed and then incubated in a α MEM medium containing 10% human serum under a 5% carbon oxide gas concentration at 37°C for a week. Views immediately after thawing, and views on day 1 and day 7 after thawing is started of each specimen were observed by a microscope. Representative examples are shown in Fig. 1, Fig. 2 and Fig. 3. As shown in Fig. 1, there was no difference in specimens immediately after thawing in both groups D and C. As is apparent from Fig. 2, there is no particular change when compared with the specimen immediately after thawing in group D, while cells begin to migrate in the vicinity of tissue pieces in group C. On day 7 after culture is started, migration of cells was observed in two fragmental skin specimens (sections) in group D (refer to d-1 in Fig. 3), while migration of cells were not observed in other three fragmental skin specimens and cell death was observed (refer to d-2 in Fig. 3). In group C on day 7 after culture is started, satisfactory migration of a lot of cells from all five fragmental skin specimens was observed (refer to c-1 and c-2 in Fig. 3).

As described above, in group D, the number of migrated cells is apparently small, and in at least half of the fragmental skin specimens, cell death in dermis and no cell migration were observed. In contrast, in group C, the number of migrated cells is apparently large, and also there was no single fragmental skin specimen in which cell death was observed in dermis and no cell migration was observed.

### INDUSTRIAL APPLICABILITY

It has hitherto been quite difficult to semipermanently preserve cells unless being formed into a complete form of cells. However, it becomes possible to preserve desired cells simply and effectively by finely dividing a tissue, such as skin, into fragmental pieces, migrating cells of interest to the border or marginal area of the cells, and subsequently freeze-preserving the entire tissue pieces. Thus, the present invention is useful in life science industry, such as diagnosis or treatment using cell, and pharmaceutical industry.
While the invention has been described by way of the foregoing Examples, but is not limited to the Examples. Modifications and adjustments of the embodiments or Examples can be made without departing from the overall disclosure (including claims) of the present invention based on the basic technical idea. Also various combinations and selections of various disclosed essential elements can be made without departing from claims of the present invention. Other problems, objects and development modes of the present invention will become apparent from the all disclosed matters including the claims.

## Claims

1. A method for cryopreservation of tissue-derived cells, comprising:
dividing a tissue containing cells of interest into fragments pieces;
culturing the divided tissue pieces in a predetermined culture medium;
collecting the cultured tissue pieces;
suspending the cultured tissue pieces in a cryoprotective solution; and
freezing the resulting suspension of the tissue pieces at a temperature of -70°C or lower.

2. The method according to claim 1, wherein the tissue is a tissue sampled from a mammal, or a cultured tissue.

3. The method according to claim 1, wherein the tissue pieces are cultured for a period required for cells in the tissue to migrate to the border or marginal area of the tissue pieces.

4. The method according to claim 1, wherein the predetermined culture medium is a culture medium capable of migrating desired cells existing in the tissue to the border or marginal area of the tissue piece(s).

5. The method according to any one of claims 1 to 4, further comprising thawing the frozen suspension of the tissue piece(s), culturing the thawed tissue piece(s), and collecting cells migrated in the culture medium.

6. A method for cryopreservation of dermis-derived fibroblasts, comprising:
dividing a skin sampled from a mammal into fragments pieces;
culturing the divided skin piece(s) in a serum-containing minimal essential medium;
collecting the cultured skin pieces;
suspending the cultured skin pieces in a cryoprotective solution; and
freezing the resulting suspension of the skin pieces at a temperature of -70°C or lower.

7. The method according to claim 6, wherein the divided skin pieces have a maximum diameter of 5 mm or less.

8. The method according to claim 6, wherein the divided skin pieces have a maximum diameter of 1 mm to 0.5 mm.

9. The method according to any one of claims 6 to 8, wherein the skin pieces are cultured at a temperature of about 37°C for 5 to 10 days.

10. The method according to any one of claims 6 to 8, wherein the skin pieces are cultured at a temperature of about 37°C for about 7 days.
